# EUROPEAN PATENT SPECIFICATION

(11) **EP 4 037 738 B1**
(45) Date of publication and mention of the grant of the patent: **18.02.2026**
(21) Application number: 20765292.6
(22) Date of filing: 07.09.2020
(51) Int. Cl.: A61M 5/32

(54) **A CAP ASSEMBLY**
KAPPENANORDNUNG
ASSEMBLAGE DE CHAPEAU

(30) Priority: 01.10.2019 EP 19200666
(43) Date of publication of application: 10.08.2022
(73) Proprietor: SHL Medical AG, 6302 Zug (CH)
(72) Inventor: YAO, Jyun-An, Taoyuan City, 338 (TW); CHEN, Hsueh-Yi, Taoyuan City, 338 (TW); CHOU, Jung-Chien, Taoyuan City, 338 (TW); LEE, Chun-Yi, Taoyuan City, 338 (TW); TU, Shih, Hsun, Taoyuan City, 338 (TW)
(86) International application number: PCT/EP2020/074926
(87) International publication number: WO 2021/063634

(56) References cited:
- WO-A1-2015/187793
- WO-A1-2017/100501
- WO-A1-2018/237225

## Description

### TECHNICAL FIELD

The present invention relates to a cap assembly for a medicament delivery device and more particularly to a cap assembly having an electronic component.

### BACKGROUND

Medicament delivery devices such as auto-injectors, inhalers, or on-body devices are generally known for the self-administration of a medicament by patients without formal medical training. For example, patients suffering from diabetes or people who are undergoing an artificial fertilization procedure may require repeated injections of insulin or hormone. Other patients may require regular injections of other types of medicaments, such as a growth hormone.

Since those medicament delivery devices are designed for patients without formal medical training and operation of those medicament delivery devices might be taking place in a patients' own house, which is usually not in a place of professional health/ medical care, e.g. hospital, clinic or heath centres, there is a demand for automatically recording every single delivery operation that has been taken by the user. The record can help the user to track his/ her medicament intake or as the basis of an alarm as the next operation reminder; the record can also help a medical doctor or a health care provider to track the compliance of the user regarding to the therapeutic regimen. Furthermore, to ensure users' safety of accessing/ using a medicament delivery device, there is also a demand for stopping users from accessing or using a medicament delivery device which has been used.

Electronic medicament delivery devices have been developed for allowing patients themselves to safely administer a medicament, without the need of help by health professionals, and for allowing transmission of data to the health professionals. Data is generally transmitted by an electronic component that is powered by a battery integrated within the device or through a wired connection by an external power source. Integrated batteries and wired connections present several disadvantages such as drainage of power if the device has a long shelf life.

US2002/096543A1 discloses a cap for an injection pen, which cap is arranged with an electronic component configured to detect the removal of the cap. However, US2002/096543A1 detects cap removal by detecting the presence or absence of the metal injection needle or a resonance circuit attached on the needle hub; which arrangement of the electronic component has to be powered on all the time, so that changes could be detected. Since a medicament delivery device might be stored for a long time, e.g. a couple of months or a year, before delivery to a user, power consumption could be significant and would therefore require a more powerful and higher cost battery. Other examples can be found in WO2018/237225, WO2015/187793, and WO2017/100051.

### SUMMARY

An object of the present invention is to provide a low power consumption and reliable cap assembly and more particularly for a disposable medicament delivery device, which avoids the problems of the prior art.

In the present disclosure, when the term "distal" is used, this refers to the direction pointing away from the dose delivery site. When the term "distal part/end" is used, this refers to the part/end of the delivery device, or the parts/ends of the members thereof, which under use of the medicament delivery device is/are located furthest away from the dose delivery site. Correspondingly, when the term "proximal" is used, this refers to the direction pointing to the dose delivery site. When the term "proximal part/end" is used, this refers to the part/end of the delivery device, or the parts/ends of the members thereof, which under use of the medicament delivery device is/are located closest to the dose delivery site.

Further, the term "longitudinal", "longitudinally", "axially" or "axial", refers to a direction extending from the proximal end to the distal end and along the device or components thereof in the direction of the longest extension of the device and/or component.

Similarly, the terms "traverse", "transversal", "transversally" refers to a direction generally perpendicular to the longitudinal direction.

An object of this invention is to provide a simple and reliable cap assembly for a medicament delivery device with an electronic component, which electronic component will only be activated once the cap assembly has been removed from the medicament delivery device.

According to an aspect of the invention, the object is achieved by a robust and reliable cap assembly according to claim 1.

There is hence provided a cap assembly adapted to be removably attached to a medicament delivery device, the cap assembly comprises: a longitudinally extending cap body configured to be removably attached to a proximal end of the medicament delivery device; an electronic component associated with the cap body; a power source associated with the cap body and configured for powering the electronic component; a resilient member associated with the cap body and configured to establish an electrical connection between the power source and the electronic component; wherein the cap body comprises a movable member configured to interact with the resilient member and with a component of the medicament delivery device such that when the cap body is attached to the medicament delivery device, the resilient member is prevented to establish the electrical connection between the power source and the electronic component.

According to one embodiment, the electronic component comprises a processor.

According to one embodiment, the electronic component comprises at least a memory, a clock, a communication unit, an indicator and/ or a sensor.

According to one embodiment, the memory can be a random access memory (RAM), a read-only memory (ROM), an erasable programmable read-only memory (EPROM), or an electrically erasable programmable read-only memory (EEPROM) or a Flash memory, such as a compact Flash memory.

According to one embodiment the communication unit can be a short-range communication unit, such like RFID, NFC, infra-red, ZigBee, Bluetooth.

According to one embodiment the communication unit can be a long-range communication unit, such like 3G, 4G, CAT-M1, NB-IoT, LoRa, Sigfox, 5G, GPRS.

According to one embodiment the communication unit can provide a wireless or wired communication.

According to one embodiment the indicator can be an acoustic indicator, such like a speaker or a buzzer.

According to one embodiment the indicator can be a visual indicator, such like an e-ink display, a LCD display or a LED light emitter.

According to one embodiment the indicator can be a haptic indicator, such like a vibrator.

According to one embodiment the sensor can be an orientation sensor, such like a accelerometer or a gyroscope.

According to one embodiment the sensor can be an environment condition sensor, such like a temperature sensor, vibration sensor or contact sensor.

According to one embodiment the power source is a coin sized battery or a capacitor.

According to one embodiment the power source is one battery or a set of at least two batteries stacked on top of one another or arranged end-to-end such that an edge of one battery is in contact with an edge of another battery.

According to one embodiment the power source is a rechargeable battery.

According to one embodiment the power source is a solar power supply, a capacitance-based power supply, a bio-active power supply that produces electricity by breaking down organic materials.

According to one embodiment the power source is control component which includes an energy harvester configured to harvest energy from at least one of an interrogation signal and a removal operation of the cap assembly from the medicament delivery device.

According to one embodiment the energy harvester is configured to harvest energy from a collateral device.

According to one embodiment the energy harvester connected to an electromagnetic generator.

According to one embodiment the energy harvester connected to piezoelectric material element.

According to one embodiment, the resilient member is a spring arm, a compression spring and/or a torsion spring or a combination thereof.

According to one embodiment, the resilient member is a flexible ribbon or a flexible wire or a sponge or a rubber stem.

According to one embodiment, the resilient member is made of a conductive material.

According to one embodiment, the resilient member is coated with a conductive material layer on its outer surface.

According to one embodiment, the resilient member comprises a fixed end which is integral to or fixedly connected to the cap body and a free end which is releasably connected to the movable member.

According to one embodiment, the resilient member is arranged in a tensioned configuration when the cap assembly is attached to the medicament delivery device; and is arranged in a relaxed configuration when the cap assembly is detached from the medicament delivery device.

According to one embodiment, the resilient member is configured to fixedly connect to the electronic component at its fixed end and selectively connect to the power source at its free end.

According to one embodiment, the resilient member is configured to fixedly connect to the power source at its fixed end and selectively connect to the electronic component at its free end.

According to one embodiment, the movable member is configured to interact with the resilient member and retain the resilient member in the tensed configuration when the cap assembly is attached to the medicament delivery device.

According to one embodiment, the movable member is a flexible arm comprising a radial inwardly protruding holding member configured to prevent the resilient member from establishing the electrical connection between the power source and the electronic component, and wherein the flexible arm is radially movable in relation to the cap body.

According to one embodiment, the flexible arm extends longitudinally in relation to the cap body and the holding member is arranged on a free end of the flexible arm.

According to one embodiment, the flexible arm extends laterally in relation to the cap body and the holding member is arranged on a free end of the flexible arm.

According to one embodiment, the cap assembly further comprises a blocking member associated with the cap body, and wherein the blocking member is configured to prevent the resilient member from establishing the electrical connection between the power source and the electronic component.

According to one embodiment, the resilient member comprises a fixed end which is integral to or fixedly connected to the cap body and a free end which is releasably connected to the blocking member.

According to one embodiment, the movable member is axially movable in relation to the cap body and is configured to bias the resilient member for aligning with the blocking member when the movable member interacts with the component of the medicament delivery device.

According to one embodiment, the blocking member comprises a first receiving portion configured to receive the free end of the resilient member before the medicament delivery device is coupled to the cap assembly in an assembling process.

According to one embodiment, the blocking member comprises a second receiving portion configured to receive the free end of the resilient member once the medicament delivery device is coupled to the cap assembly in an assembling process.

According to one embodiment, the movable member is an insulation resilient sheet.

According to one embodiment, the movable member is fixedly attached to the component of the medicament delivery device once the cap assembly has been assembled to the medicament delivery device.

According to one embodiment, the movable member is adhered to the component of the medicament delivery device once the cap assembly has been assembled to the medicament delivery device.

According to one embodiment, the resilient member is configured to irreversibly decouple from the movable member once the cap assembly is removed from the medicament delivery device.

According to one embodiment, a medicament delivery device comprising the cap assembly described above is provided.

According to one embodiment, the medicament delivery device may be an injection device, an on-body device, an inhalation device, a nasal sprayer or medical sprayer.

According to one embodiment, the medicament delivery device comprises a user accessible outer shell; which is the component of the medicament delivery device that is configured to interact with the movable member.

According to one embodiment, the user accessible outer shell can be a housing of the medicament delivery device or a mouthpiece of an inhalation device.

According to one embodiment, the medicament delivery device comprises an axial movable delivery member cover which is the component of the medicament delivery device that is configured to interact with the movable member.

According to one embodiment, the axial movable delivery member cover is configured to cover a medicament delivery member of the medicament delivery device.

According to one embodiment, the medicament delivery device comprises an actuation element; which is the component of the medicament delivery device that is configured to interact with the movable member.

According to one embodiment, the actuation element is configured to actuate a medicament delivery operation of the medicament delivery device.

According to one embodiment, the actuation element can be an actuation button.

According to one embodiment, the medicament delivery device comprises a safety member; which is the component of the medicament delivery device that is configured to interact with the movable member.

According to one embodiment, the medicament delivery device comprises a spray nozzle; which is the component of the medicament delivery device that is configured to interact with the movable member.

Other aspects, features, and advantages will be apparent from the summary above, as well as from the description that follows, including the figures and the claims.

Generally, all terms used in the claims are to be interpreted according to their ordinary meaning in the technical field, unless explicitly defined otherwise herein. All references to "a/an/the element, apparatus, component, means, etc. are to be interpreted openly as referring to at least one instance of the element, apparatus, component, means, etc.", unless explicitly stated otherwise.

### BRIEF DESCRIPTION OF THE DRAWINGS

The specific embodiments of the inventive concept will now be described, by way of example, with reference to the accompanying drawings, in which:
Fig. 1 displays a cap assembly attached to the proximal end of a medicament delivery device.
Fig. 2 displays the cap assembly of Fig. 1.
Fig. 3 displays a cap assembly attached to a distal end of a medicament delivery device.
Fig. 4 displays the further detail of the cap assembly of Fig. 3.
Fig. 5 displays a base of a cap assembly with a power source.
Fig. 6 displays a base of a cap assembly with a power source and an electronic component.
Fig. 7A-7B displays a resilient member of a cap assembly in a first embodiment of the cap assembly.
Fig. 8 displays a movable member of the cap assembly in the first embodiment of the cap assembly.
Fig. 9 displays a blocking member of the cap assembly in the first embodiment of the cap assembly.
Fig. 10 displays parts of the cap assembly in the first embodiment of the cap assembly.
Fig. 11A-11C displays the first embodiment of the cap assembly in different stages.
Fig. 12 displays parts of the cap assembly in a second embodiment of the cap assembly.
Fig. 13 displays a circuit used between the electronic component and the power source in the second embodiment of the cap assembly.
Fig. 14 displays a movable member of the cap assembly in the second embodiment of the cap assembly.
Figs. 15A-15B display the second embodiment of the cap assembly in different stages.
Figs. 16A-16B display a movable member with an alternative structure in the second embodiment of the cap assembly.
Figs. 17A-17B display the movement of the movable member according to Fig. 16A-16B.
Figs. 18A-18B display the movable member of the cap assembly in a third embodiment of the cap assembly when the cap assembly is attached to a medicament delivery device.
Figs. 19A-19B display the cap assembly in the third embodiment of the cap assembly when the cap assembly is detached from a medicament delivery device.

### DETAILED DESCRIPTION

The present application is directed to a cap assembly for a medicament delivery device and will now be described more fully hereinafter with reference to the accompanying drawings, in which exemplifying embodiments are shown. The cap assembly may, however, be embodied in many different forms and should not be construed as limited to the embodiments set forth herein; rather, these embodiments are provided by way of example so that this disclosure will be thorough and complete, and will fully convey the scope of the inventive concept to those skilled in the art. Like numbers refer to like elements throughout the description.

Pursuant to various embodiments, a medicament delivery device comprises a housing, a medicament delivery assembly at least partially disposed within the housing, a medicament container disposed within the housing, a delivery member connected or connectable to the medicament container, and a cap assembly being adapted to at least partially cover an end of the medicament delivery device housing. The medicament delivery device may comprise a delivery member cover at least partially disposed within the housing and associated with the medicament delivery assembly. The delivery member cover being movable between at least two positions position relative to the housing.

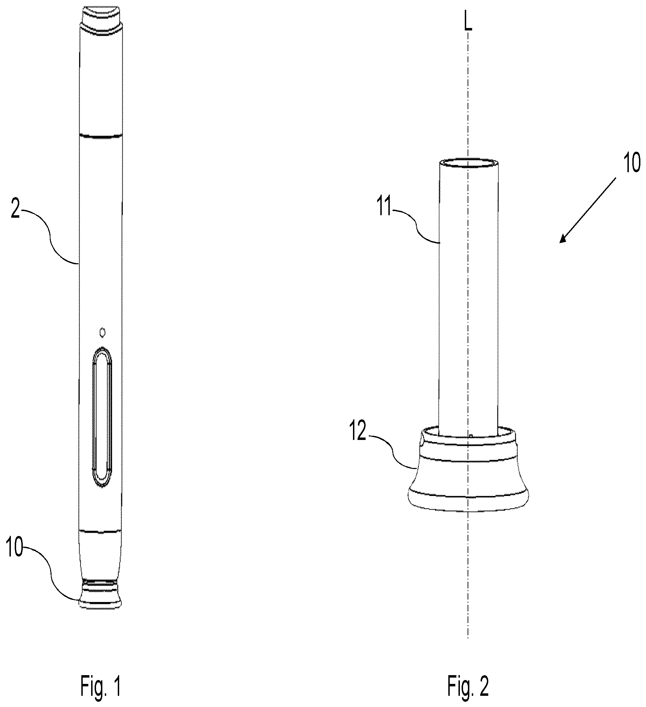

Fig. 1 illustrates a medicament delivery device (2) with an attachable cap assembly (10). The cap assembly (10) is configured to releasably cover a medicament delivery member of the medicament delivery device (2); such like a needle or a spray nozzle. As illustrated in Fig. 2, the cap assembly (10) comprises a cap body extending along a longitudinal axis (L). The cap body comprises a proximal body portion (12) and a distal body portion (11). In a preferred embodiment, the proximal body portion (12) is configured to be arranged outside of the medicament delivery device (2) when the cap assembly (10) is attached to the medicament delivery device (2), so that a user is able to grip on the outer surface of the proximal body portion (12) and remove the cap assembly (10). The distal body portion (11) is, preferably arranged inside the medicament delivery device (2), when the cap assembly (10) is attached to the medicament delivery device (2). The distal body portion (11), in a preferred embodiment, is configured to surround the medicament delivery member of the medicament delivery device (2). The distal body portion (11) may also comprise a delivery member shield remover having e.g. a gripping arm; so that if the medicament delivery member is covered by a delivery member shield, the distal body portion (11) may facilitate the user to remove the delivery member shield.

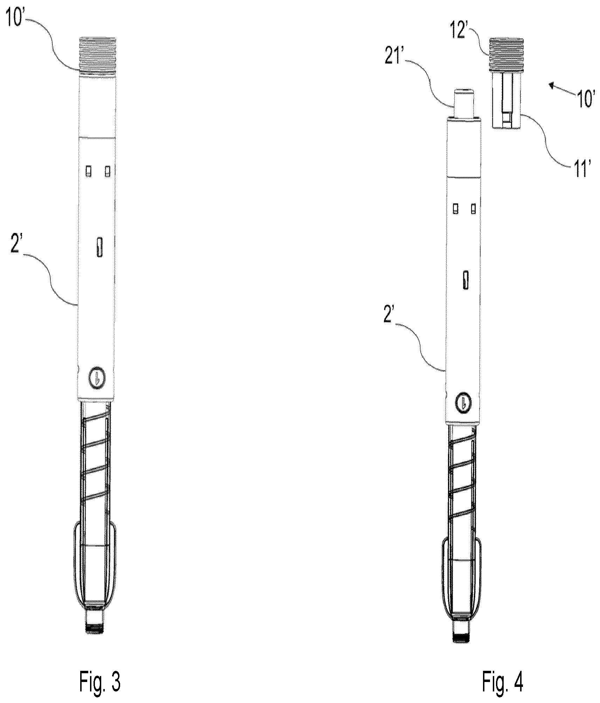

Figs. 3-4 illustrates a medicament delivery device (2') with an attachable cap assembly (10'). The longitudinal extending cap assembly (10') is arranged on the distal end of the medicament delivery device (2'), and is configured to removably cover a component (21') of the medicament delivery device (2'), such as an actuation button or a safety pin for preventing a user of the medicament delivery device (2') to access the actuation button or the safety pin before removing the cap assembly (10'). The cap assembly (10') comprises a cap body having a distal body portion (12') and a proximal body portion (11'). The distal body portion (12') is configured to be accessed by a user, so that the user can remove the cap assembly (10') by gripping the distal body portion (12'). The proximal body portion (11') is preferably arranged inside the medicament delivery device (2'), and is configured to surround the component (21') of the medicament delivery device.

The cap assembly (10; 10') further comprises a base (13); a power source (15); such like a coin size battery; an electronic component (14), preferably as a printed circuit board (PCB); a resilient member (18; 18'; 18"), which is of a conductive material; and a movable member (16; 16'; 16").

Although the power source is shown and described as comprising one battery, it is also feasible that in another embodiments may be used at least two batteries stacked on top of one another or arranged end-to-end such that an edge of one battery is in contact with an edge of another battery. The battery may be of a rechargeable type. In other embodiments, the power source can be provided to an electronic component by a source other source such as: a solar power supply, a capacitance-based power supply, a bio-active power supply that produces electricity by breaking down organic materials.

In some implementations, the power source may be a control component which includes an energy harvester configured to harvest energy from at least one of an interrogation signal and a removal operation of the cap assembly (10; 10') from the medicament delivery device (2; 2'). The control component may include a boost converter configured to increase a voltage level of an energy supplied by the energy harvester. The control component can be an ultra-low power e.g. in the µW to nW IO power range platform chip. For example, the cap assembly (10;10') can be configured to harvest energy from a collateral device and to transmit data to the collateral device. The cap assembly (10; 10') may be configured to capture RF signals generated by the collateral device and to convert these RF signals to electric signals, boosting the electric signals to feed one or more components of the cap assembly with electric energy. The cap assembly (10; 10') can then transmit data to the collateral device such as data date and time of removal of the cap assembly (10; 10') from the medicament delivery device (2; 2'). The collateral device may be a cellular/smart phone, a computer, a network appliance, or the like, or a combination thereof. The energy harvester is connected to an electromagnetic generator in another embodiment; for example, a coil. The component of the medicament delivery device (21; 21'; 21") may further be arranged with a magnet, which the magnet is placed in a surrounding area of the electromagnetic generator when the cap assembly (10; 10') is attached to the medicament delivery device (2; 2'). The removal operation of the cap assembly (10; 10') from the medicament delivery device (2; 2') causes the movement of the magnet, therefore a change of flux is provided on the electromagnetic generator, which results in induced electric energy on the electromagnetic generator based on Faraday's Law. In an alternative embodiment, the cap assembly (10; 10') may further comprise a piezoelectric material element connected to the energy harvester, which piezoelectric material element is arranged to be deformed by the removal operation of the cap assembly (10; 10') from the medicament delivery device (2; 2').

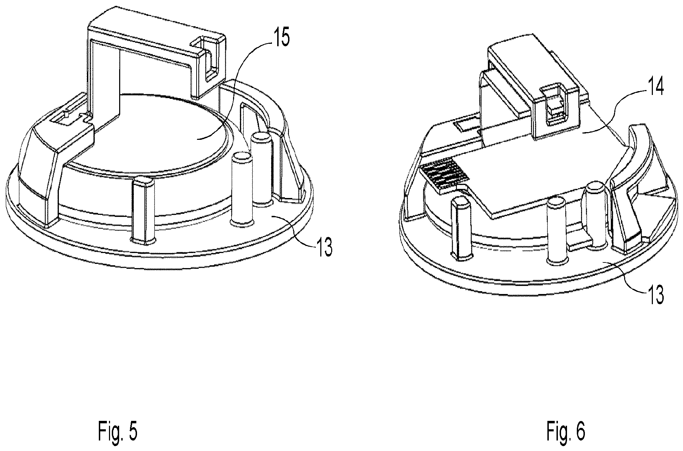

As illustrated in Fig. 5-6, the base (13) is configured to support the power source (15) and the electronic component (14). The base (13) can be integral or attachable with the body portion (12; 12') of the cap assembly (10; 10').

The electronic component (14) comprises at least two electrodes and is configured to connect to the power source (15) and form a close loop circuit. The electronic component (14) is arranged to contact with the power source (15) permanently with one electrode and the other electrode is remained free of contact with the power source (15) at an initial state. Since one of the electrode is not contact with the power source (15), there is no electrical connection between the electronic component (14) and the power source (15).

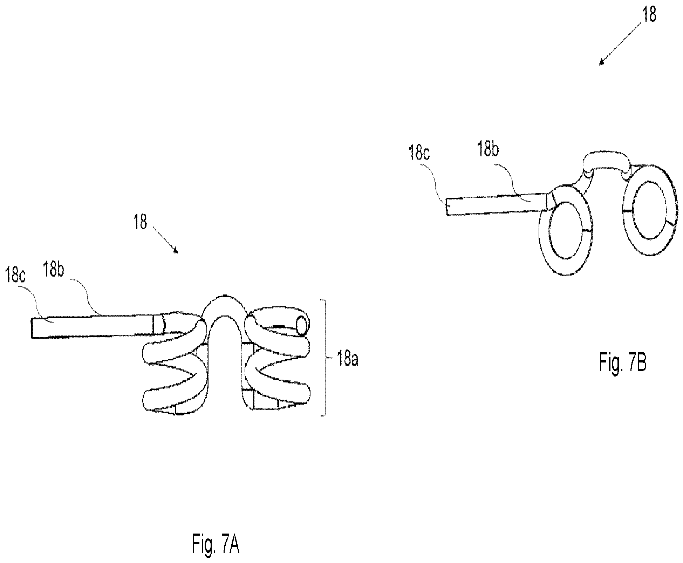

The resilient member (18) as shown in Fig 7A-7B, comprises a compression portion (18a) and a torsion portion (18b). The compression portion (18a) is configured to axially move/compress in relation to the cap body (10, 10') along the axis (L); and the torsion portion (18b) is configured to transversally move in relation to the axis (L). The torsion portion (18b) comprises a free end (18c). The compression portion (18a) is arranged on the base (13) and is in contact with one electrode of the electronic component (14), so that th compression portion (18a) is a fixed end of the resilient member (18); the torsion portion (18b) is the free end of the resilient member (18).

The movable member (16) is arranged with the compression portion (18a) of the resilient member (18) at a first end (16a) and configured to interact with a component (21; 21'; 21") of the medicament delivery device (2; 2') at a second end (16b); and is configured to move axially in relation to the cap body (10; 10') along the axis (L).

The component (21; 21'; 21") of the medicament delivery device (2; 2') that interacts with the movable member (16) is a component capable of blocking the axial movement of the movable member (16) against the axial distally biasing force from the compression portion (18a) of the resilient member (18). The component of the medicament delivery device may be, for example, an user accessible outer shell, a delivery member cover, a spray nozzle, a mouthpiece, a safety pin or an actuation button of the medicament delivery device.

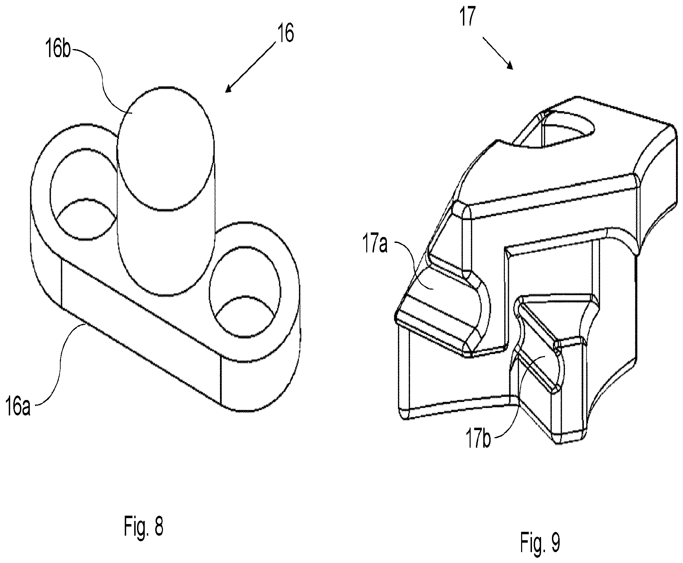

The cap assembly (10; 10') in this embodiment further comprises a blocking member (17), as shown in Fig. 9, configured to interact with the free end (18c) of the torsion portion (18b). The blocking member (17) comprises a first receiving portion (17a) and a second receiving portion (17b), which are both configured to receive the free end (18c) of the torsion portion (18b) in the different stages.

The torsion portion (18b) of the resilient member (18) is tensioned and has an accumulated biasing force when it is received in either the first receiving portion (17a) or the second receiving portion (17b). The second receiving portion (17b) is offset in relation to the first receiving portion (17a) along the path of the biasing force accumulated in the torsion portion (18b) of the resilient member (18). The free end (18c) of the torsion portion (18b) is configured to be received in the first receiving portion (17a) during an assembling process when the cap assembly (10; 10') has still not been attached to the medicament delivery device (2; 2'). Upon attaching the cap assembly to the medicament delivery device (2; 2'), the component (21; 21'; 21") of the medicament delivery device (2; 2') presses on the movable member (16), so as to press on the compression portion (18a) of the resilient member (18); so that the free end (18c) of the torsion portion (18b) will therefore be moved axially together with compression portion (18a) and aligned with the second receiving portion (17b) of the blocking member (17). Once the free end (18c) is moved out from the first receiving portion (17a), the free end (18c) starts to move transversally in relation to the axis (L)until the free end (18c) of the torsion portion (18b) is blocked and received into the second receiving portion (17b). Once a user removes the cap assembly (10; 10') from the medicament delivery device (2; 2'), the compression portion (18a) of the resilient member (18) is allowed to axially extend in relation to the axis (L), whereby the free end (18c) moves out from the second receiving portion (17b). Since the first receiving portion (17a) and the second receiving portion (17b) are offset in relation to each other, the free end (18c) will then no longer be blocked by any portion of the blocking member (18) and is free to move transversally in relation to axis (L).

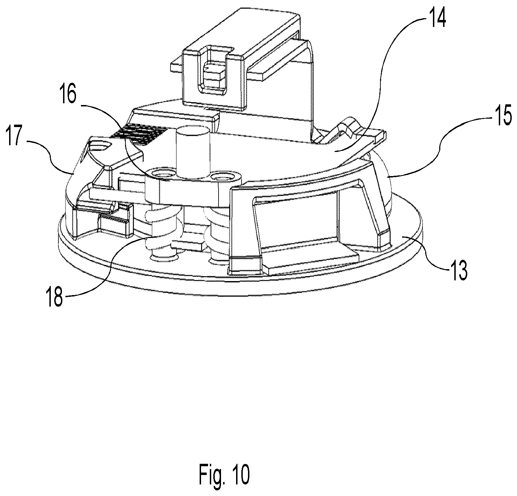

As shown in Fig. 10, the electronic component (14) is connected to the power source (15) with one electrode; and connected to the resilient member (18) with the other electrode. Thus, only when the free end (18c) of the torsion portion (18b) of the resilient member (18) is free to move transversally in relation to the axis (L) and contact with the power source (15), an electrical connection between the electronic component (14) and the power source (15) is thereby established.

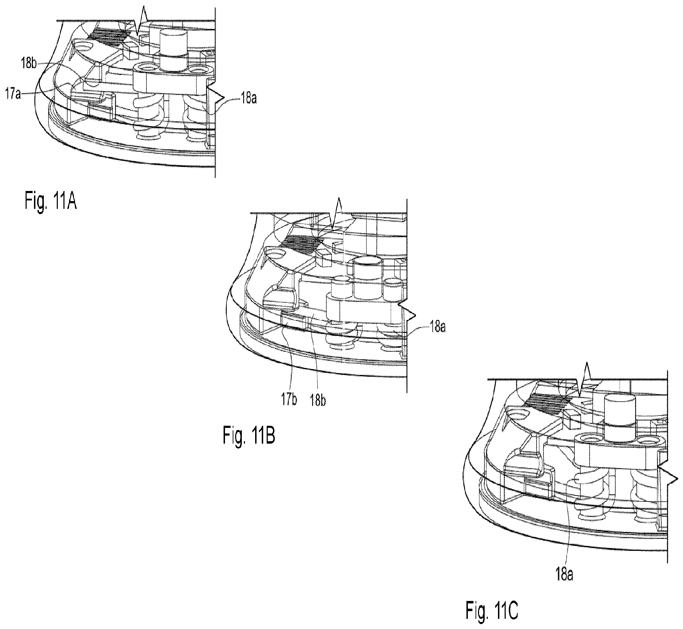

Figs. 11A-11C illustrate the above described embodiment in different stages. Fig. 11A shows an assembling process stage in which the cap assembly (10; 10') is still detached from the medicament delivery device (2; 2'). In this stage, the compression portion of the resilient member (18) is relaxed and the free end (18c) of the torsion portion (18b) of the resilient member (18) is received in the first receiving portion (17a) of the blocking member (17) and is tensioned. Fig. 11B illustrates that the cap assembly (10; 10') has been attached to the medicament delivery device (2; 2'). In this stage, the compression portion (18a) of the resilient member (18) is compressed, and the free end (18c) of the torsion portion (18b) of the resilient member (18) is received in the second receiving portion (17b) of the blocking member (17) and is still tensioned. Fig. 11C illustrates the stage when the user of the medicament delivery device (2; 2') removes the cap assembly (10; 10') from the medicament delivery device (2; 2'). In this stage, the compression portion (18a) of the resilient member (18) is relaxed again, and forces the torsion portion (18b) of the resilient member (18) out from the second receiving portion (17b). Due to the offset between the first receiving portion (17a) and the second receiving portion (17b), the free end of the torsion portion (18b) will not be blocked by any portion of the blocking member (17). Once the free end (18c) is moved by the tensioning force accumulated in the torsion portion (18b) of the resilient member (18), the free end contacts with the power source (15) and establish an electrical connection between the power source (15) and the electronic component (14).

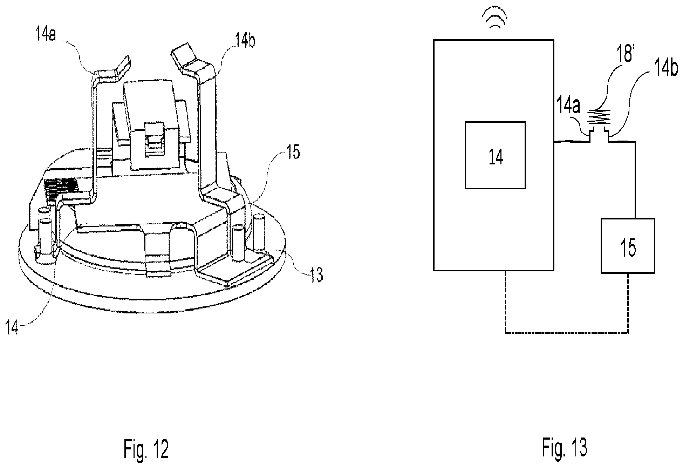

Fig. 12 illustrates another embodiment. The arrangement of the base (13) and the power source (15) is the same as described above. The electronic component (14) in this embodiment comprises a first electrode (14a) and a second electrode (14b). The electrical connection between the power source (15) and the electronic component (14) is established through the connection between the first electrode (14a) and the second electrode (14b) as shown in Fig. 13.

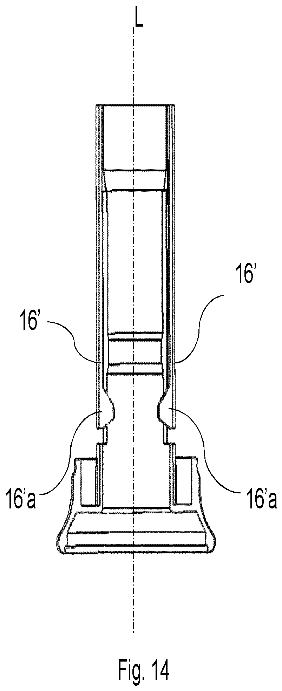

The movable member (16') in this embodiment, as shown in Fig. 14, is arranged on the distal body portion (11) of the cap body as shown in Fig 2. Moreover, the movable member is indeed arranged on the proximal body portion (11') of the cap body (10) as shown in Fig. 4.

The movable member (16') comprises a pair of flexible arms which extend longitudinally along the longitudinal axis (L) and wherein each arm has a fixed end and a free end. The fixed ends are integral to or fixedly arranged to the cap body. Each of the flexible arms respectively comprises a radial inwardly protruding holding member (16'a) on its free end. The radial inwardly protruding holding member (16a') in a preferred embodiment has a wedge shape. The free ends of the flexible arms are configured such that they can flex radially outwards in relation to the longitudinal axis (L) of the cap body.

The flexible arms are configured to interact with a component (21; 21'; 21") of the medicament delivery device (2; 2'). The component (21; 21'; 21") of the medicament delivery device (2; 2') is configured to surround the outer surface of the flexible arms (16', 16') such that the radial outward movement of the free ends of the flexible arms is restricted when the cap assembly (10; 10') is attached to the medicament delivery device (2; 2'). The free ends are arranged such that there is a predetermined distance to the first and second electrodes.

The resilient member (18') in this embodiment is preferably a compression spring and is configured to axially move along the longitudinal axis (L). The resilient member (18') is compressed and arranged between a ledge on the inner surface of the cap body and the holding member (16'a) once the cap assembly (10; 10') has been attached to the medicament delivery device (2; 2').

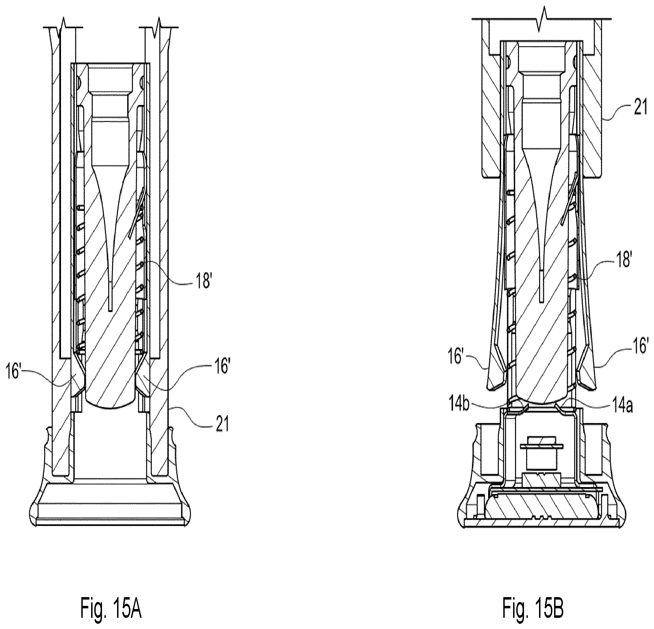

As shown in Fig. 15A, before a user removes the cap assembly (10; 10') from the medicament delivery device (2; 2'), the component (21) of the medicament delivery device interacts with the movable member (16') and prevents the free ends of the movable member (16') to flex radially outwards, so that the resilient member (18') is compressed. Once the user of the medicament delivery device (2; 2') removes the cap assembly (10; 10') from the medicament delivery device (2; 2'), as shown in Fig. 15B, the component (21) of the medicament delivery device (2; 2') is no longer interacting with the movable member (16'), so that the free ends of the movable member are able to move radially outward, whereby the resilient member (18') will move axially beyond the holding member (16'a), and make contact with the first electrode (14a) and the second electrode (14b), such that the electrical connection between the electronic component (14) and the power source (15) is thereby established.

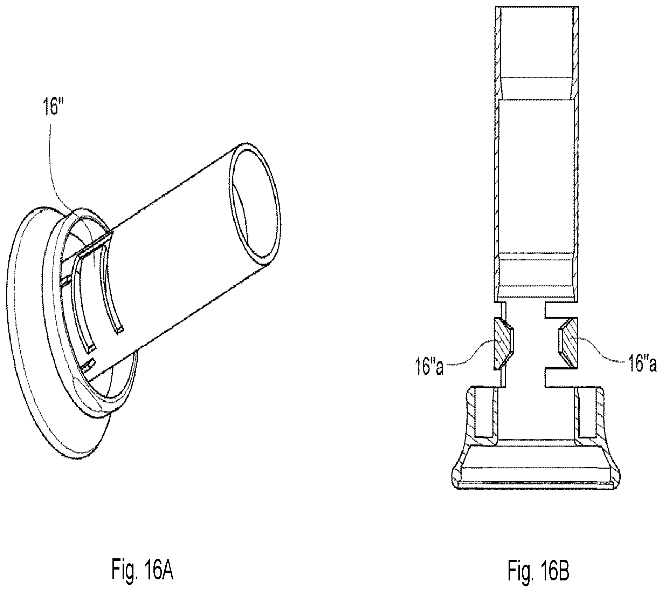

The movable member (16") in this embodiment can be modified with the structure as shown in Figs. 16A-16B, wherein the movable member (16") extends transversally in relation to the longitudinal axis (L). The movable member (16") comprises a flexible arm which has a fixed end integral or fixed to the cap body and a free end. The free end comprises a radial inwardly protruding holding member (16"a), preferably is a ledge with a wedge shape edge.

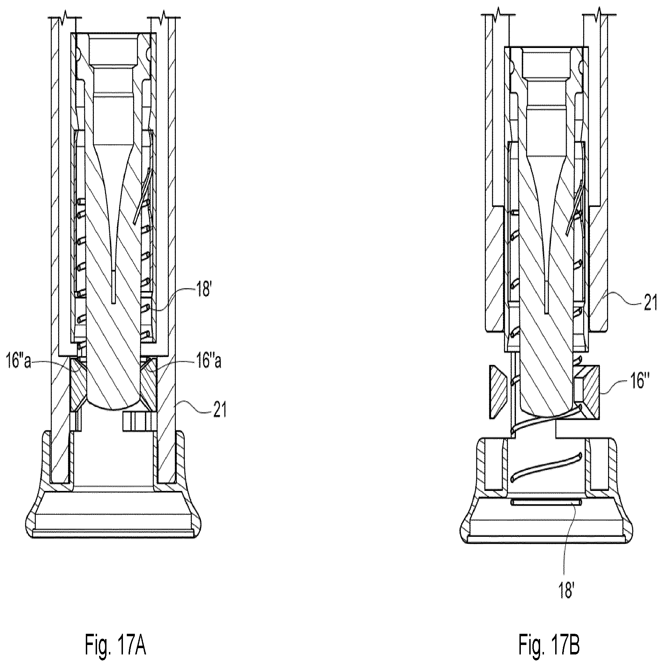

As shown in Fig. 17A-17B, the component (21) of the medicament delivery device (2; 2') is configured to interact with the movable member (16") when the cap assembly (10; 10') is attached to the medicament delivery device (2; 2'). The resilient member (18') is arranged in its compressed configuration and arranged between a ledge of the cap body and the radial inwardly protruding holding member (16"a). When the cap assembly (10; 10') is detached from the medicament delivery device (2; 2'), the flexible arm (16") is allowed to move radially outwards under the biasing force of the resilient member (18'). The resilient member (18') will then establish the electrical connection between the electronic component and the power source (15) as described above.

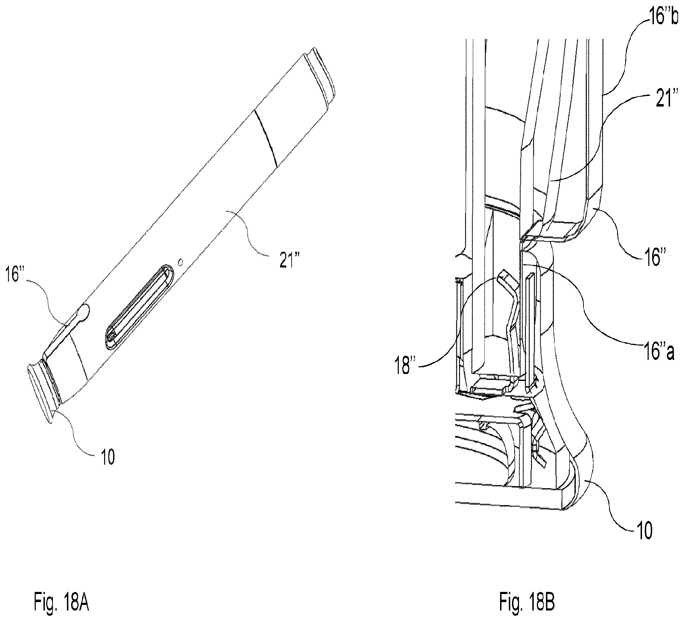

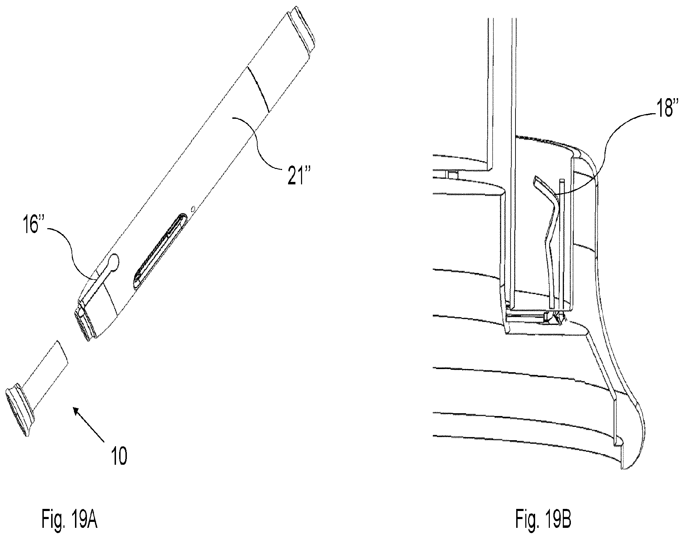

Figs. 18A-18B and Figs. 19A-19B illustrate a further embodiment. The power source (15) and the electronic component (14) in this embodiment are the same as in the described embodiments above. The electronic component (14) is connected to the power source (15) with only one electrode at the initial state; the electrical connection between the power source (15) and the electronic component (14) need to be established through the resilient member (18"), which is connected to both another electrode of the electronic component (14) and the power source (15).

As shown in Figs. 18A-18B, the cap assembly (10; 10') comprises the movable member (16") and a resilient member (18"). The movable member (16") is a resilient insulation sheet having a holding member (16"a) and an extending member (16"b). The resilient member (18") comprises a fixed end, which is fixed to the cap body, and a free end, which is radially movable in relation to the cap body. The free end of the resilient member (18") is tensioned and preferably flexes radially inward during the assembling process. The holding member (16"a) of the movable member (16") is placed adjacent to the free end of the resilient member (18"), such that a radial outward movement of the free end of the resilient member (18") is prevented.

When the cap assembly (10; 10') is attached to the medicament delivery device (2), the extending member (16"b) of the movable member (16") is configured to be fixed to the component (21") of the medicament delivery device (2), preferably by an adhesive feature.

In a preferred embodiment, the fixed end of the resilient member (18") is also connected to the power source (15). Since the holding member (16"a) of the movable member (16") insulates the free end of the resilient member (18") with the electrode of the electronic component (14), so an electrical connection between the electronic component (14) and the power source (15) is prevented.

As shown in Figs. 19A-19B, since the extending member (16"b) of the movable member (16") is fixed to the component (21") of the medicament delivery device (2), once the cap assembly (10; 10') is removed from the medicament delivery device (2), the movable member (16") will also be moved with the medicament delivery device (2). The holding member (16"a) of the movable member (16") is thereby removed from the free end of the resilient member (18"). The free end of the resilient member (18") will then flex radially outward under the resilient force of the resilient member (18"), and contact with the electrode of the electronic component (14); such that the electrical connection between the electronic component (14) and the power source (15) is established.

In all embodiments after the cap assembly (10; 10') has been removed from the medicament delivery device (2; 2'), the resilient member (18; 18'; 18") is allowed to move from its tensioned/ compressed configuration to its relax configuration; and cannot be moved back into its tensioned/ compressed configuration without a manufacturing tool. The resilient member (18; 18'; 18") is therefore no longer effectively impacted by the movement of the movable member (16; 16'; 16") to further interact with the electronic component (14) and/ or the power source (15). The resilient member (18; 18'; 18") and the movable member (16; 16'; 16") are thereby irreversibly decoupled from each other.

Since the resilient member (18; 18'; 18") is thereby irreversibly decoupled from the movable member (16; 16'; 16") after the cap assembly (10; 10') has been removed from the medicament delivery device (2; 2'), the electrical connection between the electronic component (14) and the power source (15), will not be broken even if the cap assembly (10; 10') is reattached to the medicament delivery device (2; 2').

Since the electrical connection between the electronic component (14) and the power source (15) will only be established once the cap assembly (10; 10') is removed from the medicament delivery device (2; 2'), there will be no power consumption during the stored stage or shipping stage of the medicament delivery device (2; 2').

After the cap assembly (10; 10') is removed from the medicament delivery device (2; 2'), the electrical connection between the electronic component (14) and the power source (15) is established. The electronic component (14) is configured to perform at least one function or a combination thereof such as recording the cap removal event in a memory of the electronic component (14); providing an indication to the user of the medicament delivery device (2; 2') that the cap has been removed; sending a signal to a collateral device, to the cloud, to a remote computing device or to a combination of those thereof that the cap has been removed, sensing an environment condition, communicating with an external computing device.

Since the electrical connection between the electronic component (14) and the power source (15) will not be broken even if the cap assembly (10; 10') is reattached to the medicament delivery device (2; 2'), the arrangement can also act as a tamper proof evidence of the medicament delivery device (2; 2'), such that the electronic component (14) can provide an indication to the user if the removal of the cap assembly (10; 10') has occurred before.

The inventive concept has mainly been described above with reference to a few examples. However, as is readily appreciated by a person skilled in the art, other embodiments than the ones disclosed above are equally possible within the scope of the inventive concept, as defined by the appended claims.

## Claims

1. A cap assembly (10; 10') adapted to removably attach to a medicament delivery device (2; 2'), the cap assembly (10; 10') comprises:
a longitudinally extending cap body configured to be removably attached to the medicament delivery device (2; 2');
an electronic component (14) associated with the cap body;
a power source (15) associated with the cap body and configured for powering the electronic component (14);
a resilient member (18; 18'; 18") associated with the cap body and
configured to establish an electrical connection between the power source (15) and the electronic component (14); wherein the resilient member is configured to fixedly connect to the electronic component at its fixed end and selectively connect to the power source at its free end; or the resilient member is configured to fixedly connect to the power source at its fixed end and selectively connect to the electronic component at its free end;
**characterized in that** the cap body comprises a movable member (16; 16'; 16") configured to interact with the resilient member (18; 18'; 18"), wherein the resilient member (18; 18'; 18") comprises the fixed end which is fixedly connected to the cap body and the free end which is releasably connected to the movable member; and with a component (21; 21') of the medicament delivery device such that when the cap body is attached to the medicament delivery device (2; 2'), the resilient member is prevented to establish the electrical connection between the power source (15) and the electronic component (14).

2. The cap assembly (10; 10') according to claim 1, wherein the resilient member (18; 18'; 18") is a spring arm, a compression spring and/or a torsion spring or a combination thereof.

3. The cap assembly (10; 10') according to any one of the claims 1 - 2, wherein
the movable member (16'; 16") is a flexible arm (16'; 16") comprising a radial inwardly protruding holding member (16'a; 16"a) configured to prevent the resilient member (18') from establishing the electrical connection between the power source (15) and the electronic component (14), and wherein the flexible arm (16'; 16") is radially movable in relation to the cap body.

4. The cap assembly (10; 10') according to claim 3, wherein the flexible arm (16') extends longitudinally in relation to the cap body and the holding member (16'a) is arranged on a free end of the flexible arm (16').

5. The cap assembly (10; 10') according to claim 4, wherein the flexible arm (16") extends laterally in relation the cap body and the holding member (16"a) is arranged on a free end of the flexible arm (16").

6. The cap assembly (10; 10') according to claim 1 or 2, wherein the cap assembly (10; 10') further comprises a blocking member (17) associated with the cap body, and wherein the blocking member (17) is configured to prevent the resilient member (18) from establishing the electrical connection between the power source (15) and the electronic component (14).

7. The cap assembly (10; 10') according to claim 6, wherein the resilient member (18) comprises a fixed end (18a) which is fixedly connected to the cap body and a free end (18c) which is releasably connected to the blocking member (17).

8. The cap assembly (10; 10') according to claim 7, wherein the movable member (16) is axially movable in relation to the cap body and is configured to bias the resilient member (18) for aligning the free end (18c) of the resilient member (18) with the blocking member (17) when the movable member (16) interacts with the component (21; 21') of the medicament delivery device (2; 2').

9. The cap assembly (10; 10') according to claim 8, wherein the blocking member (17) comprises a first receiving portion (17a) configured to receive the free end (18c) of the resilient member (18) before the medicament delivery device (2; 2') is coupled to the cap assembly (10; 10') in an assembling process.

10. The cap assembly (10; 10') according to claim 9, wherein the blocking (17) member comprises a second receiving portion (17b) configured to receive the free end (18c) of the resilient member (18) once the medicament delivery device (2; 2') is coupled to the cap assembly (10; 10') in an assembling process.

11. The cap assembly (10; 10') according to any one of preceding claims, wherein the resilient member (18; 18'; 18") is configured to irreversibly decouple from the movable member (16; 16'; 16") once the cap assembly (10; 10') is removed from the medicament delivery device (2; 2').

12. The cap assembly (10; 10') according to any one of preceding claims, wherein the power source (15) comprises an energy harvester configured to harvest energy from at least one of an interrogation signal and a removal operation of the cap assembly from the medicament device (2; 2').

13. The cap assembly (10; 10') according to claim 12, wherein the energy harvester is configured to harvest energy from a collateral device.

14. A medicament delivery device (2; 2') comprising a cap assembly (10; 10') according to any one of preceding claims.

15. The medicament delivery device (2; 2') according to claim 14, comprising a user accessible outer shell; which is the component (21; 21'; 21") of the medicament delivery device (2; 2') that is configured to interact with the movable member (16; 16'; 16").

16. The medicament delivery device (2; 2') according to claim 15, comprising an axial movable delivery member cover which is the component (21; 21') of the medicament delivery device (2; 2') that is configured to interact with the movable member (16; 16'; 16").

## Patentansprüche

1. Kappenbaugruppe (10; 10'), die angepasst ist, um an eine Medikamentenabgabevorrichtung (2; 2') entfernbar angebracht zu werden, wobei die Kappenbaugruppe (10; 10') umfasst:
ein sich in Längsrichtung erstreckender Kappenkörper, der konfiguriert ist, um an der Medikamentenabgabevorrichtung (2; 2') entfernbar angebracht zu werden;
eine elektronische Komponente (14), die an dem Kappenkörper angeschlossen ist;
eine Leistungsquelle (15), die an dem Kappenkörper angeschlossen und zum Versorgen der elektronischen Komponente (14) mit Leistung konfiguriert ist;
ein elastisches Element (18; 18'; 18"), das an dem Kappenkörper angeschlossen und konfiguriert ist, um eine elektrische Verbindung zwischen der Leistungsquelle (15) und der elektronischen Komponente (14) herzustellen; wobei das elastische Element konfiguriert ist, um mit der elektronischen Komponente an ihrem festen Ende fest verbunden zu werden und mit der Leistungsquelle an ihrem freien Ende wahlweise verbunden zu werden; oder das elastische Element konfiguriert ist, um mit der Leistungsquelle an ihrem festen Ende fest verbunden zu werden und mit der elektronischen Komponente an ihrem freien Ende wahlweise verbunden zu werden; **dadurch gekennzeichnet, dass** der Kappenkörper ein bewegliches Element (16; 16'; 16") umfasst, das konfiguriert ist, um mit dem elastischen Element (18; 18'; 18") zusammenzuwirken, wobei das elastische Element (18; 18'; 18") das feste Ende umfasst, das mit dem Kappenkörper und dem freien Ende, das an dem beweglichen Element lösbar verbunden ist; und mit einer Komponente (21; 21') der Medikamentenabgabevorrichtung derart fest verbunden ist, dass, wenn der Kappenkörper an die Medikamentenabgabevorrichtung (2; 2') angebracht wird, das elastische Element daran gehindert wird, die elektrische Verbindung zwischen der Leistungsquelle (15) und der elektronischen Komponente (14) herzustellen.

2. Kappenbaugruppe (10; 10') nach Anspruch 1, wobei das elastische Element (18; 18'; 18") ein Federarm, eine Druckfeder und/oder eine Torsionsfeder oder eine Kombination davon ist.

3. Kappenbaugruppe (10; 10') nach einem der Ansprüche 1 bis 2, wobei
das bewegliche Element (16'; 16") ein flexibler Arm (16'; 16") ist, umfassend ein radial nach innen vorstehendes Halteelement (16'a; 16"a), das konfiguriert ist, um zu verhindern, dass das elastische Element (18') die elektrische Verbindung zwischen der Leistungsquelle (15) und der elektronischen Komponente (14) herstellt, und wobei der flexible Arm (16'; 16") in Bezug auf den Kappenkörper beweglich radial beweglich ist.

4. Kappenbaugruppe (10; 10') nach Anspruch 3, wobei sich der flexible Arm (16') in Längsrichtung in Bezug auf den Kappenkörper erstreckt und das Halteelement (16'a) an einem freien Ende des flexiblen Arms (16') angeordnet ist.

5. Kappenbaugruppe (10; 10') nach Anspruch 4, wobei sich der flexible Arm (16") seitlich in Bezug auf den Kappenkörper erstreckt und das Halteelement (16"a) an einem freien Ende des flexiblen Arms (16") angeordnet ist.

6. Kappenbaugruppe (10; 10') nach Anspruch 1 oder 2, wobei die Kappenbaugruppe (10; 10') ferner ein Sperrelement (17), das an dem Kappenkörper angeschlossen ist, umfasst, und wobei das Sperrelement (17) konfiguriert ist, um zu verhindern, dass das elastische Element (18) die elektrische Verbindung zwischen der Leistungsquelle (15) und der elektronischen Komponente (14) herstellt.

7. Kappenbaugruppe (10; 10') nach Anspruch 6, wobei das elastische Element (18) ein festes Ende (18a) umfasst, das mit dem Kappenkörper fest verbunden ist, und ein freies Ende (18c), das mit dem Sperrelement (17) lösbar verbunden ist.

8. Kappenbaugruppe (10; 10') nach Anspruch 7, wobei das bewegliche Element (16) in Bezug auf den Kappenkörper axial beweglich ist und konfiguriert ist, um das elastische Element (18) vorzuspannen, zum Ausrichten des freien Endes (18c) des elastischen Elements (18) mit dem Sperrelement (17), wenn das bewegliche Element (16) mit der Komponente (21; 21') der Medikamentenabgabevorrichtung (2; 2') zusammenwirkt.

9. Kappenbaugruppe (10; 10') nach Anspruch 8, wobei das Sperrelement (17) einen ersten Aufnahmeabschnitt (17a) umfasst, konfiguriert ist, um das freie Ende (18c) des elastischen Elements (18) aufzunehmen, bevor die Medikamentenabgabevorrichtung (2; 2') mit der Kappenbaugruppe (10; 10') in einem Montageprozess gekoppelt wird.

10. Kappenbaugruppe (10; 10') nach Anspruch 9, wobei das Sperrelement (17) einen zweiten Aufnahmeabschnitt (17b) umfasst, der konfiguriert ist, um das freie Ende (18c) des elastischen Elements (18) aufzunehmen, sobald die Medikamentenabgabevorrichtung (2; 2') mit der Kappenbaugruppe (10; 10') in einem Montageprozess gekoppelt wird.

11. Kappenbaugruppe (10; 10') nach einem der vorstehenden Ansprüche, wobei das elastische Element (18; 18'; 18") konfiguriert ist, um von dem beweglichen Element (16; 16'; 16") irreversibel entkoppelt zu werden, sobald die Kappenbaugruppe (10; 10') von der Medikamentenabgabevorrichtung (2; 2') entfernt wird.

12. Kappenbaugruppe (10; 10') nach einem der vorstehenden Ansprüche, wobei die Leistungsquelle (15) einen Energiegewinner umfasst, der konfiguriert ist, um Energie aus mindestens einem von einem Abfragesignal und einem Entfernungsvorgang der Kappenbaugruppe von der Medikamentenvorrichtung (2; 2') zu gewinnen.

13. Kappenbaugruppe (10; 10') nach Anspruch 12, wobei der Energiegewinner konfiguriert ist, um Energie aus einer zusätzlichen Vorrichtung zu gewinnen.

14. Medikamentenabgabevorrichtung (2; 2'), umfassend eine Kappenbaugruppe (10; 10') nach einem der vorstehenden Ansprüche.

15. Medikamentenabgabevorrichtung (2; 2') nach Anspruch 14, umfassend eine für den Benutzer zugängliche Außenhülle; die die Komponente (21; 21'; 21") der Medikamentenabgabevorrichtung (2; 2') ist, die konfiguriert ist, um mit dem beweglichen Element (16; 16'; 16") zusammenzuwirken.

16. Medikamentenabgabevorrichtung (2; 2') nach Anspruch 15, umfassend eine axial bewegliche Abgabeelementabdeckung, die die Komponente (21; 21') der Medikamentenabgabevorrichtung (2; 2') ist, die konfiguriert ist, um mit dem beweglichen Element (16; 16' 16") zusammenzuwirken.

## Revendications

1. Ensemble capuchon (10 ; 10') adapté pour se fixer de manière amovible à un dispositif de délivrance de médicament (2 ; 2'), l'ensemble capuchon (10 ; 10') comprend :
un corps de capuchon s'étendant longitudinalement conçu pour être fixé de façon amovible au dispositif de délivrance de médicament (2 ; 2') ;
un composant électronique (14) associé au corps de capuchon ;
une source de puissance (15) associée au corps de capuchon et conçue pour alimenter en puissance le composant électronique (14) ;
un élément élastique (18 ; 18' ; 18") associé au corps de capuchon et conçu pour établir une connexion électrique entre la source de puissance (15) et le composant électronique (14) ; dans lequel l'élément élastique est conçu pour se relier fixement au composant électronique au niveau de son extrémité fixe et se relier sélectivement à la source de puissance au niveau de son extrémité libre ; ou l'élément élastique est conçu pour se relier fixement à la source de puissance au niveau de son extrémité fixe et se relier sélectivement au composant électronique au niveau de son extrémité libre ; **caractérisé en ce que** le corps de capuchon comprend un élément mobile (16 ; 16' ; 16") conçu pour interagir avec l'élément élastique (18 ; 18' ; 18"), dans lequel l'élément élastique (18 ; 18' ; 18") comprend l'extrémité fixe qui est reliée fixement au corps de capuchon et l'extrémité libre qui est reliée de manière libérable à l'élément mobile ; et à un composant (21 ; 21') du dispositif de délivrance de médicament de telle sorte que lorsque le corps de capuchon est fixé au dispositif de délivrance de médicament (2 ; 2'), l'élément élastique est empêché d'établir la connexion électrique entre la source de puissance (15) et le composant électronique (14).

2. Ensemble capuchon (10 ; 10') selon la revendication 1, dans lequel l'élément élastique (18 ; 18' ; 18") est un bras de ressort, un ressort de compression et/ou un ressort de torsion ou une combinaison de ceux-ci.

3. Ensemble capuchon (10 ; 10') selon l'une quelconque des revendications 1 à 2, dans lequel
l'élément mobile (16' ; 16") est un bras flexible (16' ; 16") comprenant un élément de maintien (16'a ; 16"a) radial faisant saillie vers l'intérieur conçu pour empêcher l'élément élastique (18') d'établir la connexion électrique entre la source de puissance (15) et le composant électronique (14), et dans lequel le bras flexible (16' ; 16") est mobile en sens radial par rapport au corps de capuchon.

4. Ensemble capuchon (10 ; 10') selon la revendication 3, dans lequel le bras flexible (16') s'étend longitudinalement par rapport au corps de capuchon et l'élément de maintien (16'a) est agencé sur une extrémité libre du bras flexible (16').

5. Ensemble capuchon (10 ; 10') selon la revendication 4, dans lequel le bras flexible (16") s'étend latéralement par rapport au corps de capuchon et l'élément de maintien (16"a) est agencé sur une extrémité libre du bras flexible (16").

6. Ensemble capuchon (10 ; 10') selon la revendication 1 ou 2, dans lequel l'ensemble capuchon (10 ; 10') comprend en outre un élément de blocage (17) associé au corps de capuchon, et dans lequel l'élément de blocage (17) est conçu pour empêcher l'élément élastique (18) d'établir la connexion électrique entre la source de puissance (15) et le composant électronique (14).

7. Ensemble capuchon (10 ; 10') selon la revendication 6, dans lequel l'élément élastique (18) comprend une extrémité fixe (18a) qui est reliée fixement au corps de capuchon et une extrémité libre (18c) qui est reliée de manière libérable à l'élément de blocage (17).

8. Ensemble capuchon (10 ; 10') selon la revendication 7, dans lequel l'élément mobile (16) est mobile axialement par rapport au corps de capuchon et est conçu pour solliciter l'élément élastique (18) pour un alignement de l'extrémité libre (18c) de l'élément élastique (18) avec l'élément de blocage (17) lorsque l'élément mobile (16) interagit avec le composant (21 ; 21') du dispositif de délivrance de médicament (2 ; 2').

9. Ensemble capuchon (10 ; 10') selon la revendication 8, dans lequel l'élément de blocage (17) comprend une première partie de réception (17a) conçue pour recevoir l'extrémité libre (18c) de l'élément élastique (18) avant que le dispositif de délivrance de médicament (2 ; 2') ne soit accouplé à l'ensemble capuchon (10 ; 10') dans un procédé d'assemblage.

10. Ensemble capuchon (10 ; 10') selon la revendication 9, dans lequel l'élément de blocage (17) comprend une seconde partie de réception (17b) conçue pour recevoir l'extrémité libre (18c) de l'élément élastique (18) une fois que le dispositif de délivrance de médicament (2 ; 2') est accouplé à l'ensemble capuchon (10 ; 10') dans un procédé d'assemblage.

11. Ensemble capuchon (10 ; 10') selon l'une quelconque des revendications précédentes, dans lequel l'élément élastique (18 ; 18' ; 18") est conçu pour se désaccoupler de manière irréversible de l'élément mobile (16 ; 16' ; 16") une fois que l'ensemble capuchon (10 ; 10') est retiré du dispositif de délivrance de médicament (2 ; 2').

12. Ensemble capuchon (10 ; 10') selon l'une quelconque des revendications précédentes, dans lequel la source de puissance (15) comprend un collecteur d'énergie conçu pour collecter de l'énergie provenant d'au moins l'un parmi un signal d'interrogation et une opération de retrait de l'ensemble capuchon du dispositif de médicament (2 ; 2').

13. Ensemble capuchon (10 ; 10') selon la revendication 12, dans lequel le collecteur d'énergie est conçu pour collecter de l'énergie provenant d'un dispositif accessoire.

14. Dispositif de délivrance de médicament (2 ; 2') comprenant un ensemble capuchon (10 ; 10') selon l'une quelconque des revendications précédentes.

15. Dispositif de délivrance de médicament (2 ; 2') selon la revendication 14, comprenant une coque externe accessible par un utilisateur ; qui est le composant (21 ; 21' ; 21") du dispositif de délivrance de médicament (2 ; 2') qui est conçu pour interagir avec l'élément mobile (16 ; 16' ; 16").

16. Dispositif de délivrance de médicament (2 ; 2') selon la revendication 15, comprenant un couvercle d'élément de délivrance déplaçable axialement qui est le composant (21 ; 21') du dispositif de délivrance de médicament (2 ; 2') qui est conçu pour interagir avec l'élément mobile (16 ; 16' ; 16").
